# EUROPEAN PATENT SPECIFICATION

(11) **EP 1 106 190 B1**
(45) Date of publication and mention of the grant of the patent: **10.09.2003**
(21) Application number: 00126419.1
(22) Date of filing: 05.12.2000
(51) Int. Cl.: A61L 29/04, C08L 25/08, C08L 23/02

(54) **Medical tubing**
Medizinischer Schlauch
Tubulure médicale flexible

(30) Priority: 09.12.1999 JP 34979099
(43) Date of publication of application: 13.06.2001
(73) Proprietor: Nipro Corporation, Kita-ku, Osaka-shi, Osaka-fu, 531-8510 (JP)
(72) Inventor: Nizuka, Takeshi, Kita-ku, Osaka-shi, Osaka-fu 531-8510 (JP); Mitani, Hideki, Kita-ku, Osaka-shi, Osaka-fu 531-8510 (JP); Shimada, Mamoru, Kita-ku, Osaka-shi, Osaka-fu 531-8510 (JP)
(74) Representative: Albrecht, Thomas, Dr.

(56) References cited:
- WO-A-99/45980
- US-A- 5 356 709
- US-A- 5 863 977
- DATABASE WPI Section Ch, Week 198538 Derwent Publications Ltd., London, GB; Class A17, AN 1985-234391 XP002163438 & JP 60 153866 A (SUMITOMO CHEM IND KK), 13 August 1985 (1985-08-13)

## Description

### Field of the Invention

The present invention relates to a medical tubing, and especially relates to a medical tubing which can constitute a part of medical equipment such as infusion circuits, blood circuits, and the like.

### Background of the Invention

Tubes formed mainly from a soft polyvinyl chloride (PVC) containing diethylhexylphthalate (DEHP) or the like as a plasticizer have been used for a long period of time as medical tubing and, more particularly, have been used as conjunction tubes or connecting tubes of medical equipment such as infusion circuits, blood circuits, and the like. However, PVC is a homopolymer of vinyl chloride which contains a chlorine atom. Therefore, when products formed from the polymer are incinerated, there is a possibility that a harmful substance, dioxin, is generated depending on the conditions of incineration. Further, there is a possibility that DEHP contained in PVC as a plasticizer is eluted into a drug liquid charged in PVC products or into blood passed through the PVC products, and be introduced in a human body. Furthermore, there is a problem that a drug such as nitroglycerin that is required to be accurately injected is adsorbed by the tubes and a desired amount of the drug is not administered.

It is supposed that harmful dioxins are not generated when a PVC product is incinerated at a high temperature. A general small-sized incinerator is not satisfactory for such incineration because the incinerator cannot provide a high temperature. A large-sized incinerator to meet this requirement is desired but a high cost is required to install such an incinerator.

Further, DEHP contained in PVC products as a plasticizer is a low molecular weight compound and it is expected that it is easily eluted from PVC products and introduced into a human body. In particular, when a drug containing ethanol or polyethylene glycol as a dissolving agent or blood is contained in a bag formed from PVC containing DEHP, or is passed through a tube formed from the polymer, its elution is remarkable. For this reason, PVC products not containing a plasticizer have been investigated. One example thereof is a PVC product obtained by copolymerization with an ethylene-vinyl acetate copolymer according to a specific method. However, this PVC product also contains chlorine atoms and its cost is very high. Thus, such a product can not generally be used. Further, in the case of a drug such as nitroglycerin that requires a precise injection being contained in the bag, the problem of drug adsorption has not yet been solved.

In view of the above, an infusion tube formed from a very low density polyethylene having a density of 0.89 g/cm³ or less has been proposed as a medical tubing for intravenous injection of nitroglycerin (Japanese Patent Examined Publication No. Hei 1-48775). This tube adsorbs a very small amount of nitroglycerin, and therefore an intended amount of the drug can be administered. However, there is a physical problem that although the tube has sufficient flexibility for use as a medical tubing, the tube does not withstand squeezing by an infusion pump when an accurate flow rate is required.

The drug adsorption property of an infusion tube formed from polyvinyl chloride or polybutadiene has been discussed (M. Gerard Lee, American Journal of Hospital Pharmacy, Vol. 43, Aug. p.1945-1950, 1986). A tube made of polybutadiene also has a very small adsorption property with respect to a drug such as nitroglycerin, and therefore is suitable for administration of such a drug. However, since a double bond still remains in a polymer chain, storage conditions or processing conditions are restricted, or a stabilizer must be used, in order to prevent a change in the physical properties of the tube during storage for a long period of time, or to prevent gelation during molding and processing of the tube. Thus, such a tube does not always satisfy safety requirements when used as a medical tube.

Further, to sterilize a tube made of 1,2-polybutadiene with γ-ray radiation, it is necessary to add a phosphorus-based antioxidant (Japanese Patent Unexamined Publication No. Hei 5-98075). Such a composition is not preferable as a medical tubing from this standpoint. Also, there is a problem that this medical tubing does not withstand squeezing by an infusion pump.

Furthermore, a resin composition comprising polybutadiene and a styrene-isoprene-styrene block copolymer is proposed as a composition suitable for medical products such as medical tubing, soft catheters, sheets and the like (Japanese Patent Unexamined Publication No. Hei 6-184360). This resin composition has very excellent flexibility, and therefore is put into practical use as an infusion tube for a pump. However, because it cannot withstand deterioration of portions squeezed by the pump, a silicone resin is coated on its surface. Moreover, because a styrene-isoprene-styrene block copolymer is unstable, there is a problem that the tube turns yellow during storage for a long period of time or in γ-ray sterilization.

Also, a drug administration tube comprising a blend of polyethylene and a styrene-based elastomer or olefin-based elastomer has been proposed (Japanese Patent Examined Publication No. Hei 2-31990). Since drug adsorption is small by this tube, the intended amount of drug can be administered. However, because an elastomer is added to polyethylene, rigidity of a molded tube decreases and there is a problem that the tube does not withstand the squeezing action of a pump.

WO 99/45980 discloses medical devices comprising an aromatic vinyl/α-olefin random copolymer. JP60153866 discloses styrene/propylene resins having a styrene content of 1-3 mol.% of styrene.

In view of the above situation, the present inventors variously investigated medical tubing formed of a material not containing a chloride atom or a plasticizer for the purpose of obtaining the medical tubing having low drug adsorption, resistance to squeezing by a pump, a possibility of undergoing γ-ray sterilization, and a flexibility necessary for clinical usage. The inventors found that a random copolymer of an a-olefin-based monomer and a styrene-based monomer achieves the expected purpose of the present invention.

### Brief Description of the Drawing

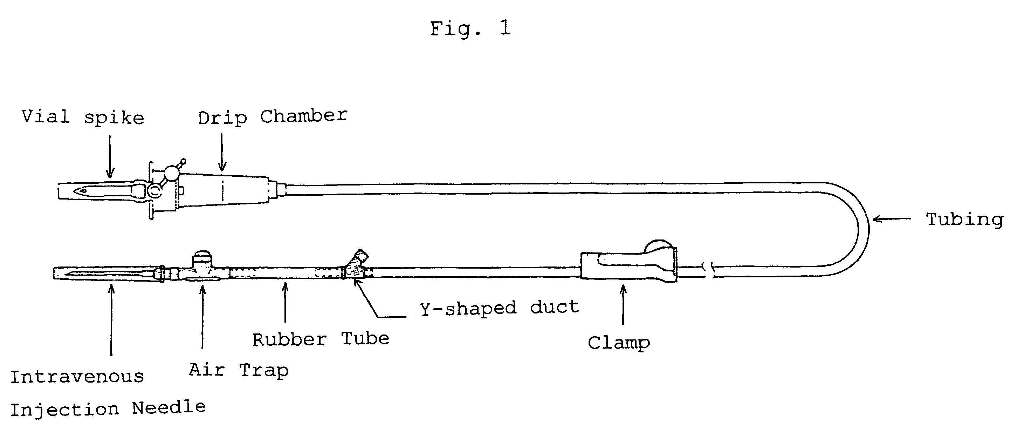

Fig. 1 is a view showing one embodiment of the use of the medical tubing of the present invention.

### Summary of the Invention

The present invention relates to a plasticizer-free medical tubing for administering an injection solution containing nitroglycerin comprising a tube formed from a random copolymer consisting of 80-90 mole% ethylene and 20-10 mole% styrene and wherein the melt flow ratio (200°C) of said random copolymer is 0.3-10 g/10 mins.

The random copolymer comprising an olefin-based monomer and a styrene-based monomer useful in the present invention is a random copolymer comprising ethylene and styrene.

In the present invention, the random copolymer comprising ethylene and styrene can be a random copolymer copolymerized with another monomer such as a monomer containing a glycidyl group, amino group, dimethylamino group, hydroxyl group, carboxyl group, ester group, ether group or isocyanate group, or a maleic anhydride. The copolymerization proportion thereof is within a range that achieves the purpose of the present invention.

The copolymerization ratio of the random copolymer of the present invention is one where the ethylene is 80-90 mol% and the styrene is 20-10 mol%. If the styrene is less than 5 mol%, hardness of the tube is increased and transparency thereof is reduced. Further, if it exceeds 50 mol%, the elasticity necessary for a medical tubing cannot be obtained.

A specific example of the present invention is a medical tubing containing a random copolymer of ethylene and styrene. The random copolymer of ethylene and styrene is preferably one in which the ethylene content is 80-90 mol% and the styrene content is 20-10 mol%.

The ethylene-styrene random copolymer used in the present invention is a random copolymer of ethylene and styrene and optionally, contains another olefin monomer if necessary.

The random copolymer means a copolymer in which a proportion of a block or blocks comprising two or more continuous units derived from the α-olefin based monomer or styrene based monomer is 10% or less, and preferably, 1% or less with respect to the amount of a copolymer derived from both monomers. The content of a block or blocks consisting of two or more continuous units derived from the styrene-based monomer may be determined by ¹³C-NMR.

Styrene-ethylene-butylene-styrene block copolymer (SEBS), styrene-isoprene-styrene block copolymer (SIS), styrene-butadiene-styrene block copolymer (SBS) and the like have conventionally been known as a copolymer of an olefin such as ethylene with a vinyl aromatic compound such as styrene. These copolymers have styrene block portions and the olefin block portions, respectively. On the contrary, the ethylene-styrene random copolymer of the present invention is one in which ethylene and styrene are randomly copolymerized as mentioned above, and a low styrene content region does not have a block portion of styrene in the structure of the copolymer. As a result, this random copolymer substantially differs from the above-mentioned styrene-based elastomer such as SEBS.

The melt flow ratio (200°C, MFR) of the random copolymer of the present invention is 0.3-10 g/10mins. The melting point is 100°C or lower.

The random copolymer of an olefin-based monomer and a styrene-based monomer used in the present invention is synthesized by conventional methods. There are, for example, methods such as radical polymerization, ion polymerization, and the like. A high-pressure radical polymerization method is described in Japanese Patent Unexamined Publication No. Hei 1-113447. It is disclosed that the monomers are simultaneously or stepwise contacted with each other and polymerized in the presence of a radical polymerization initiator, a chain transfer agent and, if necessary, an auxiliary agent under conditions of a polymerization pressure of 500-4000 Kg/cm² and a polymerization temperature of 50-400°C in a reaction vessel.

Further, a method of producing the above-mentioned random copolymer using a metallocene catalyst is disclosed in Japanese Patent Unexamined Publication No. Hei 10-273544 and Japanese Patent No. 2,623,070. The metallocene catalyst used in those methods is a metallocene catalyst conventionally used as a single site catalyst, and a metallocene catalyst similar to such a catalyst. In particular, a catalyst comprising a metallocene compound of a transition metal (transition metal compound) and an organic aluminum oxy compound and/or an ionized ionic compound is preferably used.

The medical tubing of the present invention can be used as a means for transporting a liquid in medical equipment such as an infusion circuit or a blood circuit, and can forcedly transport a liquid by a pump such as infusion pump. One example thereof is shown in Fig. 1. The pump presses the tube from the periphery thereof and transports a drug solution to an objective site while squeezing the tubing. As a result, an accurate flow quantity is transported to a body from an infusion bottle or bag. However, in order to withstand squeezing for a long period of time, it is necessary for the tube to have a sufficient elasticity. The medical tubing in the present invention can provide the required elasticity for use in the equipment. The medical equipment is not limited to the above-mentioned infusion circuit and blood circuit, but includes equipment necessary for various liquid transportation in the medical field.

The medical tubing of the present invention is formed from a random copolymer comprising ethylene units and styrene units, and does not contain a plasticizer. Therefore, unlike PVC tube containing a plasticizer, the tubing has a characteristic that a plasticizer does not elute from the tubing. Further, the tubing is suitable for administrating drugs because the copolymer does not adsorb oil-soluble drugs such as nitroglycerin. The plasticizer referred to herein means a compound added to conventional thermoplastic polymers such as polyvinyl chloride, and which has a possibility of affecting the human body, such as phthalates, e.g., di(2-ethylhexyl)phthalate, di(isodecyl) phthalate, or adipates, e.g., dioctyl adipate.

The medical tubing of the present invention is obtained by general extrusion molding. If necessary, additives such as antioxidants, lubricants, or the like may be added to the random copolymer. The shape, diameter, length and other parameters of the tube can be selected according to the intended purpose of use, and are not particularly limited.

The obtained tubing is joined with another part such as a drip chamber used in infusion administration, and then packed in a wrapping and sterilized. Ethylene oxide gas sterilization, γ-ray sterilization or electron ray sterilization can be selected as the sterilization method. The medical tubing of the present invention does not contain butadiene units or the like that require additives when conducting γ-ray or electron ray sterilization. It is therefore considered that γ-ray or electron ray sterilization is possible even in the state of not containing additives such as phosphorus-based antioxidants, e.g., bis (2,6-di-t-butyl-4-methylphenyl)pentaerythritol diphosphite or bis(2,4-di-t-butylphenyl)pentaerythritol diphosphite or the like.

### Examples

The present invention is described below in detail using Examples and Comparative Examples.

In the Examples and Comparative Examples, each test item was measured as follows.

### Measurement of color tone and transparency:

After gamma ray irradiation (25 kgy), visually observed.

### Eluent test by sterilized infusion set standard:

A tube portion of 10 g was cut in about a 1 cm length, and boiled in 100 ml distilled water for 30 minutes. Distilled water was added to accurately make 100 ml, thereby preparing a test liquid. Simultaneously, only distilled water was boiled for 30 minutes to prepare a blank test liquid.

<pH> 20 ml of each of the test liquid and blank test liquid was collected. 1.0 g of potassium chloride was dissolved in water to make 1000 ml, and 1.0 ml thereof was added to each of the test liquid and the blank test liquid. pH change of the liquids was measured by a pH measurement method defined in Japanese Pharmacopoeia . When the pH difference was 2.0 or less, it was considered to be acceptable.

<Heavy metal> 10 ml of a test liquid was collected, and tested according to Method No. 1 of the Heavy Metal Test Method defined in Japanese Pharmacopoeia. For a blank test liquid, 2.0 ml of lead standard liquid was added and tested in the same manner. When color was not dark as compared with the blank test liquid, it was considered to be acceptable.

<Potassium permanganate reducing substance> 10 ml of a test liquid was collected in a stoppered triangular flask, 20.0 ml of 0.002 mol/liter potassium permanganate solution and 1 ml of diluted sulfuric acid were added thereto, and the flask was sealed. The flask was shaken and then allowed to stand for 10 minutes, followed by titration with 0.01 mol/liter sodium thiosulfate solution (5 drops of indicator, starch reagent). Separately, the same procedure was conducted using 10 ml of a blank test liquid. When the difference between a test liquid and the blank test liquid in the amount of 0.002 mol/liter potassium permanganate solution consumed was 2.0 ml or less, it was considered to be acceptable.

<Evaporation residue> 10 ml of a test liquid was evaporated to dryness on a water bath, and the residue was dried at 105°C for 1 hour. If its weight at that time was 1.0 mg or less, it was considered to be acceptable.

### Nitroglycerin adsorption property:

60 ml of a nitroglycerin injection (effective component:50 mg/100 ml, Millisrol® injection, manufactured by Nippon Kayaku Co.) was poured into 1 liter of a physiological saline defined in Japanese Pharmacopoeia (manufactured by Otsuka Pharmaceutical Co.), followed by slowly stirring. Sampling was immediately conducted by an injection syringe equipped with an injection needle to prepare a blank sample. A tube of an infusion set was closed with a clamp for controlling flow rate, and a bottle needle of a drip chamber was pierced into the rubber plug of an infusion container. A lower half portion of the drip chamber was filled with the solution by pumping the drip chamber. The clamp for controlling flow rate was gradually released to fill the inside of the tube with the solution, and the tube was attached to an infusion pump FP-2001, manufactured by Nissho Co. Flow rate was set to 36 ml/hr, the clamp for controlling flow rate was opened, the switch was on, and then infusion was initiated. Sampling of the solution which flowed out from the end of the tube was chronologically conducted, and the concentration was measured with high performance liquid chromatography. Infusion was conducted for 180 hours, and sampling was conducted every 5 minutes for the first 60 minutes and every 15 minutes after then.
Column: CAPCELL PAK® C18 SG120Å 5 µm φ4.6 mm x 250 mm manufactured by Shiseido Co.
Temperature: 30°C
Mobile phase: methanol:water=11:9
Detector: UV wavelength 210 nm
Flow rate: 0.8 ml/min

If 90% or less of the concentration was observed as compared with the blank concentration, it was considered that there is adsorption.

### Pump flow rate stability (250 ml/hr, 24 hrs):

A tube was closed with a clamp for controlling flow rate, and a bottle needle of a drip chamber was pierced into a rubber plug of a container of physiological saline defined in Japanese Pharmacopoeia (manufactured by Otsuka Pharmaceutical Co.). A lower half portion of the drip chamber was filled with the saline by pumping the drip chamber. The clamp for controlling flow rate was gradually released to fill the inside of the tube with the saline, and the tube was attached to an infusion pump FP-2001, manufactured by Nissho Co. Flow rate was set to 250 ml/hr, the clamp for controlling flow rate was opened, the switch was turned on, and infusion was initiated. The amount of physiological saline which flowed out of the top of the tube was immediately received by a measuring cylinder to measure flow rate per minute (V₀). After 24 hours, flow rate per minute (V₁) was again measured in the same manner. Flow rate change (V₁-V₀/V₀)x100(%) after 24 hours was obtained. It was considered to be acceptable if the change was within ±15%.

### Tube state after finishing pump infusion:

After testing the above pump flow rate stability, the surface state, the change in tube diameter and the presence or absence of cracks were observed on the squeezed portion of the tube.

### Example 1

An ethylene-styrene random copolymer (MFR 9 g/10mins, melting point 71°C) constituted of 15mol% styrene and 85% ethylene was extrusion molded at a melting temperature of 200°C to obtain a tube having an inner diameter of 2.7 mm and an outer diameter of 3.8 mm. Conventional plasticizer was not added.

The obtained tube was cut into a length of about 1.2 m, and passed through a clamp for controlling flow rate. A drip chamber equipped with a vial spike was connected to one end of the tube, and an intravenous needle, an air trap, an isoprene rubber tubing and a Y-shape duct were connected to the other end of the tube to obtain an infusion set as shown in Fig. 1. After sterilization of the tube with γ-ray irradiation, the color tone and transparency of the tube were measured, and an eluted material test according to the sterilized infusion set standard was conducted. And nitroglycerin adsorption property, pump flow rate stability (250 ml/hr, 24 hours) and tube state after finishing pump infusion were observed. The results obtained are shown in Table 1.

### Example 2

A tube was prepared in the same manner as in Example 1 except that an ethylene-styrene random copolymer (MFR 4 g/10mins, melting point 79°C) having a styrene content of 10 mol% and an ethylene content of 90 mol% was used in place of the ethylene-styrene random copolymer (MFR 9 g/10mins, melting point 71°C) having a styrene content of 15 mol% and an ethylene content of 85 mol%, and the same tests were conducted. The results obtained are shown in Table 1.

### Example 3

A tube was prepared in the same manner as in Example 1 except that an ethylene-styrene random copolymer (MFR 5 g/10mins, melting point 31°C) having a styrene content of 20 mol% and ethylene content of 80 mol% was used in place of the ethylene-styrene random copolymer (MFR 9 g/10mins, melting point 71°C) having a styrene content of 15 mol% and an ethylene content of 85 mol%, and the same tests were conducted. The results obtained are shown in Table 1.

### Example 4

A tube was prepared in the same manner as in Example 1 except that an ethylene-styrene random copolymer (MFR 0.5 g/10mins, melting point 68°C) having a styrene content of 15 mol% and an ethylene content of 85 mol% used in place of the ethylene-styrene random copolymer (MFR 9 g/10mins, melting point 71°C) having a styrene content of 15 mol% and an ethylene content of 85 mol%, and the same tests were conducted. The results obtained are shown in Table 1.

### Comparative Example 1

A tube (inner diameter 2.7 mm, outer diameter 3.8 mm, length 120 cm) obtained from polyvinyl chloride containing 35% by weight of di (2-ethylhexyl)phthalate as a plasticizer was subjected to the same tests as in Example 1. The results obtained are shown in Table 2

### Comparative Example 2

A tube (inner diameter 2.7 mm, outer diameter 3.8 mm, length 120 cm) obtained from syndiotactic 1,2-polybutadiene (manufactured by JSR Corporation, RB810, density 0.901 g/cm³, melting point 71°C, melt index 3 g/10mins-150°C-2160 g) was subjected to the same tests as in Example 1. The results obtained are shown in Table 2.

### Comparative Example 3

A tube (inner diameter 2.7 mm, outer diameter 3.8 mm, length 120 cm) obtained from a mixture of 80% by weight of syndiotactic 1,2-polybutadiene (manufactured by JSR Corporation, RB820, density 0.906 g/cm³, melting point 95°C, melt index 3 g/10mins-150°C-2160 g) and 20% by weight of styrene-isoprene-styrene block copolymer (manufactured by JSR corporation, SIS5000P, styrene content 15% by weight, density 0.92 g/cm³, MFR 2 g/10mins-200°C-5 kg) was subjected to the same tests as in Example 1. The results obtained are shown in Table 2.

### Comparative Example 4

A tube (inner diameter 2.7 mm, outer diameter 3.8 mm, length 120 cm) obtained from a mixed resin of 70% by weight of linear low density polyethylene (manufactured by Dow Chemical Co., PF1140, density 0.895 g/cm³) and 30% by weight of styrene-based elastomer (manufactured by Shell, KRATON® G1657, SEBS, styrene content 13% by weight, MFR 8 g/10mins-200°C-5.0 kg) was subjected to the same tests as in Example 1. The results obtained are shown in Table 2.

**[Table 1]**

| Test | Examples | | | |
|---|---|---|---|---|
| | 1 | 2 | 3 | 4 |
| Color tone, transparency | Colorless, transparent | Colorless, transparent | Colorless, transparent | Colorless, transparent |
| Eluted substance | Acceptable | Acceptable | Acceptable | Acceptable |
| Nitroglycerin adsorption property (maximum sorption ratio) | <5% | <5% | <5% | <5% |
| Pump flow rate stability (250 ml/after 24 hours) | <10% | <10% | <10% | <10% |
| Tube state after completion of pump infusion | No abnormality | No abnormality | No abnormality | No abnormality |
| Total evaluation | ο | ο | ο | ο |

**[Table 2]**

| Test | Comparative Examples | | | |
|---|---|---|---|---|
| | 1 | 2 | 3 | 4 |
| Color tone, transparency | Colorless, transparent | Yellowed | Yellowed | Colorless, transparent |
| Eluted substance | Acceptable | Acceptable | Acceptable | Acceptable |
| Nitroglycerin adsorption property (maximum sorption ratio) | 45% | <5% | <5% | <5% |
| Pump flow rate stability (250 ml/after 24 hours) | <10% | <10% | <10% | <10% |
| Tube state after completion of pump infusion | No abnormality, | Rough surface | Rough surface | Rough surface |
| Total evaluation | × | × | × | × |

The tubes of the above Examples 1-4 were colorless and transparent even after γ-ray sterilization was conducted. The results of the eluted substance test by sterilized infusion set standard were acceptable, nitroglycerin adsorption property was a maximum of 5%, pump flow rate variation was within 10%, and no abnormality was observed on the tube surface after finishing pump infusion. On the contrary, in the tube made of polyvinyl chloride containing a plasticizer, nitroglycerin was adsorbed on the tube (Comparative Example 1). In the tube comprising syndiotactic 1,2-polybutadine containing no antioxidant (Comparative Example 2) and the tube comprising a mixture of syndiotactic 1,2-polybutadiene and styrene-isoprene-styrene block copolymer (Comparative Example 3) were colored yellow after γ-ray sterilization was conducted. Further, the surface was scraped on squeezing with the pump, and many white powdered materials were generated. In the tube comprising a mixture of linear low density polyethylene and styrene-based elastomer (Comparative Example 4), the surface was also scraped on squeezing with the pump and many white powdered materials were generated.

According to the present invention, a random copolymer of an olefin-based monomer and a styrene-based monomer, not containing chlorine atoms or a plasticizer can provide a medical tubing in which an adsorption of drugs is very small. Further the tubing of the present invention can withstand to squeezing action by an infusion pump, has a possibility of undergoing γ-ray sterilization and has a flexibility necessary for clinical use.

## Claims

1. A plasticizer-free medical tubing for administering an injection solution containing nitroglycerin comprising a tube formed from a random copolymer consisting of 80-90 mole% ethylene and 20-10 mole% styrene and wherein the melt flow ratio (200°C) of said random copolymer is 0.3-10 g/10 mins.

2. The medical tubing of claim 1, wherein said random copolymer is further copolymerised with a monomer containing a glycidyl group, amino group, dimethylamino group, hydroxyl group, carboxyl group, ester group, ether group or isocyanate group, or with a maleic anhydride.

3. Use of the medical tubing of claims 1 or 2, as a means for forcedly conveying a liquid with a pump in medical equipment such as an infusion circuit or blood circuit.

4. The medical tubing of claims 1 or 2, for use in intravenous injection of nitroglycerin.

5. The medical tubing of claims 1 or 2, sterilized with a γ-ray or an electron ray.

## Patentansprüche

1. Weichmacherfreies medizinisches Schlauchmaterial zur Verabreichung einer Nitroglyzerin enthaltenden Injektionslösung, umfassend einen Schlauch, gebildet aus einem Random-Copolymeren, bestehend aus 80-90 Mol-% Ethylen und 20-10 Mol-% Styrol, wobei die Fließfähigkeit (200°C) des genannten Random-Copolymeren 0,3-10 g/10 min beträgt.

2. Medizinisches Schlauchmaterial nach Anpruch 1, **dadurch gekennzeichnet, dass** das genannte Random-Copolymere weiterhin mit einem Monomeren, enthaltend eine Glycidylgruppe, Aminogruppe, Dimethylaminogruppe, Hydroxylgruppe, Carboxylgruppe, Estergruppe, Ethergruppe oder Isocyanatgruppe, oder mit Maleinsäureanhydrid copolymerisiert worden ist.

3. Verwendung des medizinischen Schlauchmaterials nach Anspruch 1 oder 2 als Mittel zur Zwangsbeförderung einer Flüssigkeit mit einer Pumpe in einem medizinischen Gerät, wie in einem Infusionskreislauf oder Blutkreislauf.

4. Medizinisches Schlauchmaterial nach Anspruch 1 oder 2 zur Verwendung bei der intravenösen Injektion von Nitroglyzerin.

5. Medizinisches Schlauchmaterial nach Anspruch 1 oder 2, sterilisiert mit γ-Strahlen oder Elektronenstrahlen.

## Revendications

1. Tubulure médicale sans plastifiant pour l'administration d'une solution à injecter contenant de la nitroglycérine comportant un tube composé d'un copolymère statistique consistant en 80 à 90 % en mole d'éthylène et en 20 à 10 % en mole de styrène et dans laquelle l'indice de fusion (200 °C) dudit copolymère statistique est de 0,3 à 10 g/10 min.

2. Tubulure médicale selon la revendication 1, dans laquelle ledit copolymère statistique est polymérisé à nouveau avec un monomère contenant un groupe glycidylique, un groupe aminé, un groupe dimethylaminé, un groupe hydroxyle, un groupe carboxyle, un groupe ester, un groupe éther ou un groupe isocyanate, ou avec un anhydride maléique.

3. Utilisation de la tubulure médicale selon la revendication 1 ou 2, comme un moyen d'acheminer de force un liquide avec une pompe dans un équipement médical tels qu'un circuit de perfusion ou un circuit sanguin.

4. Tubulure médicale selon la revendication 1 ou 2, pour l'utilisation dans l'injection intraveineuse de nitroglycérine.

5. Tubulure médicale selon la revendication 1 ou 2, stérilisée par un rayon γ ou par un rayon d'électrons.
